# EUROPÄISCHE PATENTSCHRIFT

(11) **EP 4 380 463 B1**
(45) Veröffentlichungstag und Bekanntmachung des Hinweises auf die Patenterteilung: **04.11.2026**
(21) Anmeldenummer: 22758431.5
(22) Anmeldetag: 26.07.2022
(51) Int. Cl.: A61B 17/00, A61B 17/12, A61B 17/128

(54) **MEDIZINISCHES INSTRUMENT**
MEDICAL INSTRUMENT
INSTRUMENT MÉDICAL

(30) Priorität: 03.08.2021 DE 102021208392
(43) Veröffentlichungstag der Anmeldung: 12.06.2024
(73) Patentinhaber: Aesculap AG, 78532 Tuttlingen (DE)
(72) Erfinder: SCHÜLE, Jana, 88637 Kreenheinstetten (DE); TAN, Jian Zoing, 78532 Tuttlingen (DE); ROTHWEILER, Christoph, 78166 Donaueschingen (DE)
(74) Vertreter: Patentanwälte Ruff, Wilhelm, Beier, Dauster & Partner mbB
(86) Internationale Anmeldenummer: PCT/EP2022/070889
(87) Internationale Veröffentlichungsnummer: WO 2023/011976

(56) Entgegenhaltungen:
- EP-A2- 3 756 572
- US-A1- 2018 116 674
- US-B2- 10 905 420

## Beschreibung

Die Erfindung betrifft ein medizinisches Instrument, insbesondere zur Applikation medizinischer Clips, aufweisend eine Griffvorrichtung mit einem feststehenden Handgriff, einem beweglichen Handgriff, der relativ zu dem feststehenden Handgriff manuell zwischen einer nicht betätigten Ruhestellung und einer betätigten Arbeitsstellung beweglich ist, und mit einer Arretiereinrichtung, einen längserstreckten Schaft, dessen proximales Ende mittels der Arretiereinrichtung lösbar an der Griffvorrichtung arretiert ist, ein an einem distalen Ende des Schafts angeordnetes Instrumentenmaul, das mittels einer Betätigung des beweglichen Handgriffs zwischen einer Öffnungsstellung und einer Schließstellung beweglich ist, und aufweisend ein längserstrecktes Zug-Druck-Element, das axial beweglich in dem Schaft geführt und zur Kraft- und Bewegungsübertragung proximal mit dem beweglichen Handgriff und distal mit dem Instrumentenmaul wirkverbunden ist.

Ein derartiges medizinisches Instrument ist aus der EP 0 688 187 B1 bekannt und in Form einer medizinischen Zange zur Verwendung bei endoskopischen Eingriffen vorgesehen. Die bekannte Zange weist ein Handstück mit einem feststehenden Handgriff und einem beweglichen Handgriff auf. Zudem weist die medizinische Zange einen längserstreckten Schaft in Form eines Außenrohrs auf, das lösbar an dem Handstück arretiert ist. In dem Außenrohr ist eine Zugstange axial beweglich geführt, die einends mit einem distal an dem Außenrohr montierten Zangenmaul und andernends mit dem beweglichen Handgriff wirkverbunden ist. Das Außenrohr kann zusammen mit dem Zangenmaul und der Zugstange von dem Handstück demontiert werden. Dies insbesondere zur Reinigung und Sterilisierung.

Ferner ist aus der EP 3 756 572 A2 ein chirurgisches Instrument bekannt, das ein Gehäuse mit Griffteilen sowie eine daran auswechselbar befestigte, längserstreckte Schaftbaugruppe mit einem distal angeordneten Instrumentenmaul aufweist. Zur lösbaren Kopplung der Schaftbaugruppe mit dem Gehäuse ist ein Verriegelungssystem vorgesehen, dessen Betätigungselement und Sperrmechanik jedoch nicht an der Griffvorrichtung, sondern an der Schaftbaugruppe angeordnet sind.

Aufgabe der Erfindung ist es, ein medizinisches Instrument der eingangs genannten Art bereitzustellen, das eine verbesserte Patientensicherheit bietet.

Diese Aufgabe wird durch ein medizinisches Instrument gemäß Anspruch 1 gelöst.

Diese Aufgabe wird dadurch gelöst, dass die Arretiereinrichtung ein Betätigungselement, das zum Lösen der Arretierung manuell in eine Auslösestellung beweglich ist, und ein mit dem beweglichen Handgriff wirkverbundenes Sperrelement aufweist, das in der Arbeitsstellung des beweglichen Handgriffs in eine Sperrstellung verlagert ist, in welcher das Sperrelement die Beweglichkeit des Betätigungselements formschlüssig sperrt, und das in der Ruhestellung des beweglichen Handgriffs in eine Freigabestellung verlagert ist, in welcher die Beweglichkeit des Betätigungselements freigegeben ist. Durch die erfindungsgemäße Lösung wird ein unkontrolliertes Lösen der Arretierung in einem hierfür nicht bestimmten Zustand des medizinischen Instruments verhindert. Dabei geht die Erfindung von der Erkenntnis aus, dass ein Lösen der Arretierung insbesondere in einem betätigten Zustand des Instruments, d.h. in der Arbeitsstellung des beweglichen Handgriffs, mit nachteiligen Effekten für den Patienten einhergehen kann. Durch die erfindungsgemäße Lösung wird das Lösen der Arretierung in der betätigten Arbeitsstellung verhindert. Hierdurch werden nachteilige Effekte in der Verwendung des Instruments vermieden. Dies führt letztlich zu einer verbesserten Patientensicherheit. Vorzugsweise ist der bewegliche Handgriff zwischen seiner Ruhestellung und seiner Arbeitsstellung relativ zu dem feststehenden Handgriff schwenkbeweglich. Alternativ oder zusätzlich kann der bewegliche Handgriff relativ zu dem feststehenden Handgriff linearbeweglich sein. Vorzugsweise ist die Arretiereinrichtung in den feststehenden Handgriff integriert. Die Arretiereinrichtung dient einer lösbaren Arretierung des Schafts - und damit auch der mit dem Schaft verbundenen weiteren Komponenten, wie beispielsweise des Zug-Druck-Elements und des Instrumentenmauls - an der Griffvorrichtung. Vorzugsweise ist der Schaft lösbar form- und/oder kraftschlüssig mit der Griffvorrichtung verbunden, wobei die Arretiereinrichtung einem Lösen der Verbindung entgegenwirkt und/oder die Verbindung selbst bewirkt. Die besagte Verbindung zwischen dem Schaft und der Griffvorrichtung kann beispielsweise als Schraub-, Steck-, Rast- und/oder Klemmverbindung gestaltet sein. Der Schaft ist zwischen seinem proximalen Ende und seinem distalen Ende, vorzugsweise gerade, längserstreckt. Vorzugsweise ist der Schaft wenigstens abschnittsweise rohrförmig und/oder hohlzylindrisch. Der Schaft kann einstückig oder mehrteilig sein. In der Öffnungsstellung ist das Instrumentenmaul geöffnet. In der Schließstellung ist das Instrumentenmaul geschlossen. Vorzugsweise ist das Instrumentenmaul zwischen zwei relativ beweglichen Maulteilen gebildet, die in der Schließstellung aufeinander zubewegt und in der Öffnungsstellung voneinander wegbewegt sind. Das Instrumentenmaul ist über das Zug-Druck-Element mittelbar mit dem beweglichen Handgriff wirkverbunden. Eine Betätigung des beweglichen Handgriffs in dessen Arbeitsstellung kann entweder ein Öffnen oder ein Schließen des Instrumentenmauls bewirken, wobei letzteres bevorzugt ist. Das Zug-Druck-Element kann bei einer Betätigung des beweglichen Handgriffs auf Zug und/oder auf Druck auf das Instrumentenmaul wirken, wobei letzteres bevorzugt ist. Bei einer Wirkung auf Zug fungiert das Zug-Druck-Element als Zugelement. Bei einer Wirkung auf Druck fungiert das Zug-Druck-Element als Druckelement und/oder Schubelement. Das Zug-Druck-Element ist zwischen seinem proximalen Ende und seinem distalen Ende, vorzugsweise gerade und/oder koaxial zu dem Schaft, längserstreckt. Vorzugsweise ist das Zug-Druck-Element in radialer Richtung formschlüssig und in axialer Richtung gleitbeweglich in dem Schaft geführt. Eine Betätigung des beweglichen Handgriffs bewirkt eine Bewegung des Sperrelements in dessen Sperrstellung. Die Bewegung des Sperrelements zwischen der Freigabestellung und der Sperrstellung kann rotatorisch und/oder translatorisch sein. Die Bewegung des Betätigungselements in die Auslösestellung kann rotatorisch und/oder translatorisch sein. In der Sperrstellung blockiert das Sperrelement die Beweglichkeit des Betätigungselements in die Auslösestellung vorzugsweise unmittelbar und/oder formschlüssig. Das medizinische Instrument ist vorzugsweise zur Verwendung bei laparoskopischen Eingriffen vorgesehen. Vorzugsweise ist das medizinische Instrument beispielsweise eine medizinische Zange, Klemme, Schere oder dergleichen. Das Instrumentenmaul ist dementsprechend beispielsweise ein Zangen-, Klemmen- oder Scherenmaul oder dergleichen.

In Ausgestaltung der Erfindung sperrt das Betätigungselement in der Auslösestellung die Beweglichkeit des Sperrelements in die Sperrstellung und/oder die Beweglichkeit des beweglichen Handgriffs in die Arbeitsstellung. Hierdurch wird eine Betätigung des beweglichen Handgriffs in einem hierfür nicht vorgesehenen Zustand verhindert. Dabei geht diese Ausgestaltung der Erfindung von der Erkenntnis aus, dass eine Betätigung des beweglichen Handgriffs in der Auslösestellung des Betätigungselements, d.h. während des Lösens der Arretierung des Schafts, mit nachteiligen Effekten für den Patienten einhergehen kann. Durch diese Ausgestaltung der Erfindung wird das Betätigen des beweglichen Handgriffs während des Lösens der Arretierung verhindert. Hierdurch werden weitere nachteilige Effekte in der Verwendung des medizinischen Instruments vermieden. Zudem wird verhindert, dass das Betätigungselement in seiner Auslösestellung gesperrt wird. Dies führt letztlich zu einer nochmals verbesserten Patientensicherheit. Die Sperrung der Beweglichkeit des Sperrelements und/oder des beweglichen Handgriffs erfolgt vorzugsweise formschlüssig und/oder unmittelbar mittels des Betätigungselements.

In weiterer Ausgestaltung der Erfindung ist das Sperrelement mittels eines an der Griffvorrichtung angeordneten ersten Federelements in Richtung der Sperrstellung federbelastet vorgespannt, und - in dem arretierten Zustand des Schafts - mittels eines an dem Schaft angeordneten zweiten Federelements in Richtung der Freigabestellung federbelastet vorgespannt, wobei das zweite Federelement eine stärkere Vorspannung bewirkt als das erste Federelement. Diese besonders bevorzugte Ausgestaltung der Erfindung geht mit weiteren Vorteilen einher. Zur weiteren Erläuterung ist zwischen einer ersten Konfiguration und einer zweiten Konfiguration des medizinischen Instruments zu unterscheiden. In der ersten Konfiguration ist der Schaft - und damit auch das Zug-Druck-Element sowie das Instrumentenmaul - an der Griffvorrichtung montiert, d.h. mit derselben verbunden und/oder an derselben arretiert. In der zweiten Konfiguration ist der Schaft - und damit auch das Zug-Druck-Element sowie das Instrumentenmaul - von der Griffvorrichtung demontiert, d.h. die in der ersten Konfiguration ausgebildete Verbindung und/oder Arretierung ist gelöst. Die erste Konfiguration kann auch als Montagezustand bezeichnet werden. Die zweite Konfiguration kann auch als Demontagezustand bezeichnet werden. In dem Montagezustand wirken sowohl das erste als auch das zweite Federelement auf das Sperrelement ein. Das erste und das zweite Federelement wirken zueinander entgegengesetzt, wobei das zweite Federelement stärker dimensioniert ist als das erste Federelement. Durch die hierdurch bewirkte stärkere Vorspannung wird das Sperrelement in dem Montagezustand federbelastet in die Freigabestellung bewegt. Dies jedenfalls dann, wenn der bewegliche Handgriff seine Ruhestellung einnimmt und nicht zusätzlich auf das Sperrelement einwirkt. In dem Demontagezustand ist das zweite Federelement gemeinsam mit dem Schaft von der Griffvorrichtung gelöst, so dass die Vorspannung des Sperrelements in die Freigabestellung aufgehoben ist. Hierdurch wird das Sperrelement unter Einwirkung des ersten Federelements in die Sperrstellung verlagert. Aufgrund der Wirkverbindung des Sperrelements mit dem beweglichen Handgriff wird letzterer vorzugsweise gleichzeitig in die Arbeitsstellung verlagert. Im Ergebnis ist das Betätigungselement im Demontagezustand stets gesperrt. Hierdurch wird sichergestellt, dass das Betätigungselement während der Demontage des Schafts nicht betätigt werden kann. Dabei geht diese Ausgestaltung von der Erkenntnis aus, dass ein ungewolltes und/oder unkontrolliertes Betätigen des Betätigungselements während der Montage des Schafts nachteilige Wirkungen haben kann. Diese nachteiligen Wirkungen werden durch diese Ausgestaltung der Erfindung ausgeschlossen. Hierdurch wird eine nochmals verbesserte Patientensicherheit erreicht. Das erste Federelement ist vorzugsweise in die Griffvorrichtung integriert. Das zweite Federelement ist an dem Schaft angeordnet. Bei einer Ausgestaltung wirkt das erste Federelement und/oder das zweite Federelement unmittelbar auf das Sperrelement. Bei einer weiteren Ausgestaltung wirkt das erste Federelement und/oder das zweite Federelement mittelbar auf das Sperrelement. Beispielsweise kann das erste Federelement mittelbar über den beweglichen Handgriff auf das Sperrelement wirken. Das zweite Federelement kann beispielsweise über das Zug-Druck-Element mittelbar auf das Sperrelement einwirken.

In weiterer Ausgestaltung der Erfindung ist das Betätigungselement in Radialrichtung des Zug-Druck-Elements beweglich und das Sperrelement ist in Axialrichtung des Zug-Druck-Elements beweglich. Dies hat insbesondere konstruktive Vorteile. Zum einen kann die Sperrfunktion des Sperrelements konstruktiv besonders einfach umgesetzt werden. Beispielsweise kann das Sperrelement zum Sperren der radialen Beweglichkeit des Betätigungselements unter, über oder mit dem Betätigungselement zusammengeschoben werden. Die hierdurch bewirkte Sperrung ist besonders robust. Sofern zusätzlich eine Sperrung der Beweglichkeit des Sperrelements und/oder des beweglichen Handgriffs mittels des Betätigungselements vorgesehen ist, kann das Betätigungselement in axialer Bewegungsrichtung des Zug-Druck-Elements vor oder hinter dasselbe bewegt werden. Dies in Radialrichtung nach außen oder nach innen.

In weiterer Ausgestaltung der Erfindung ist das Betätigungselement ein in die Auslösestellung eindrückbarer Druckknopf, und das Sperrelement ist ein linear beweglicher Schieber, der in der Sperrstellung wenigstens abschnittsweise unter den Druckknopf geschoben ist. Diese Ausgestaltung der Erfindung ist konstruktiv besonders einfach umsetzbar. Gleichzeitig wird eine besonders robuste Sperrung der Beweglichkeit des Betätigungselements ermöglicht. Vorzugsweise ist der Druckknopf in der Auslösestellung wenigstens abschnittsweise vor den Schieber geschoben. Hierdurch wird eine einfache und robuste Sperrung der Beweglichkeit des Schiebers - und damit auch des mit dem Schieber wirkverbundenen beweglichen Handgriffs - erreicht. Vorzugsweise ist der Druckknopf mittels eines weiteren Federelements, das auch als drittes Federelement bezeichnet werden kann, entgegen einer Bewegung in die Auslösestellung federbelastet vorgespannt. Bei einer Ausgestaltung ist der Druckknopf in Radialrichtung des Zug-Druck-Elements beweglich. Bei einer weiteren Ausgestaltung ist der Druckknopf in Axialrichtung des Zug-Druck-Elements beweglich. Entsprechendes gilt sinngemäß für die Beweglichkeit des Schiebers. Vorzugsweise ist der Druckknopf und/oder der Schieber an dem feststehenden Handgriff beweglich gelagert.

In weiterer Ausgestaltung der Erfindung ist der bewegliche Handgriff um eine Schwenkachse schwenkbeweglich an dem feststehenden Handgriff gelagert, und das Sperrelement weist eine parallel zu der Schwenkachse orientierte Mitnehmerachse auf, die mit einer stirnendseitig in den beweglichen Handgriff eingebrachten Mitnehmernut zusammenwirkt. Hierdurch wird eine konstruktiv besonders einfache sowie besonders robuste Wirkverbindung zwischen dem beweglichen Handgriff und dem Sperrelement erreicht. Die Wirkverbindung dient einer Kraft- und Bewegungsübertragung zwischen dem beweglichen Handgriff und dem Sperrelement. Die Schwenkachse und/oder die Mitnehmerachse kann räumlich-körperlich in Form eines entsprechenden Bauelements vorhanden oder eine Achse im geometrischen Sinne sein. Vorzugsweise greift die Mitnehmerachse in Axialrichtung des Zug-Druck-Elements formschlüssig und/oder um die Schwenkachse des beweglichen Handgriffs formschlüssig in die Mitnehmernut ein. Vorzugsweise ist die Mitnehmerachse in ihrer Umfangsrichtung gleitbeweglich in der Mitnehmernut aufgenommen.

In weiterer Ausgestaltung der Erfindung wirkt der bewegliche Handgriff mittels des Sperrelements kraft- und bewegungsübertragend auf ein proximales Ende des Zug-Druck-Elements. Demnach weist das Sperrelement bei dieser Ausgestaltung der Erfindung eine besonders vorteilhafte Mehrfachfunktion auf. Zum einen dient das Sperrelement der besagten Sperrung der Beweglichkeit des Betätigungselements. Zum anderen wirkt das Sperrelement zusätzlich als mechanisches Übertragungselement zwischen dem beweglichen Handgriff und dem Zug-Druck-Element. Dies erlaubt eine weiter vereinfachte Konstruktion. Vorzugsweise ist der bewegliche Handgriff ausschließlich mittels des Sperrelements mit dem Zug-Druck-Element wirkverbunden.

In weiterer Ausgestaltung der Erfindung weist das Sperrelement einen distal angeordneten Stützabschnitt auf, an welchem das proximale Ende des Zug-Druck-Elements in Axialrichtung abgestützt ist und/oder in Radialrichtung formschlüssig aufgenommen ist. Durch die Abstützung in Axialrichtung wird eine vorteilhafte Kraft- und Bewegungsübertragung auf das Zug-Druck-Element erreicht. Durch die in Radialrichtung formschlüssige Aufnahme kann eine verbesserte Führung des Zug-Druck-Elements erreicht werden. Vorzugsweise weist der Stützabschnitt eine Formschlussprofilierung auf, beispielsweise in Form einer Einsenkung oder eines Zapfens, die zum Ausbilden des radialen Formschlusses in Axialrichtung mit dem proximalen Ende des Zug-Druck-Elements lösbar zusammengesteckt ist.

In weiterer Ausgestaltung der Erfindung weist die Arretiereinrichtung eine federbelastete Klinke auf, die in Axialrichtung formschlüssig in eine Kerbe des Schafts eingreift und mittels des Betätigungselements aus der Kerbe ausrückbar ist. Bei dieser Ausgestaltung der Erfindung ist die Arretierung als lösbare Klinke-Kerbe-Verbindung ausgeführt. Die Klinke ist mit dem Betätigungselement wirkverbunden. Die Klinke ist bei einer Ausgestaltung als ein von dem Betätigungselement separates und mit diesem kraft- und bewegungsübertragend verbundenes Bauteil ausgeführt. Bei einer weiteren Ausgestaltung ist die Klinke ein Abschnitt des Betätigungselements und insoweit einstückig mit demselben. Die Kerbe ist an dem proximalen Ende des Schafts angeordnet. Die Kerbe ist vorzugsweise als in Umfangsrichtung des Schafts umlaufende Vertiefung ausgebildet, beispielsweise als Ringnut. In der Auslösestellung des Betätigungselements ist die Klinke aus der Kerbe ausgerückt. Andernfalls ist die Klinke in die Kerbe eingerückt. Die Ein- und/oder Ausrückbewegung der Klinke ist vorzugsweise in Radialrichtung des Schafts und/oder Zug-Druck-Elements orientiert.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Seitenansicht eine Ausführungsform eines erfindungsgemäßen medizinischen Instruments mit einer Griffvorrichtung, einem lösbar an der Griffvorrichtung montierten Schaft und einem distal an dem Schaft angeordneten Instrumentenmaul,
- Fig. 2: in einer schematischen, teilweise abgeschnittenen Seitenansicht ein Detail des Schafts im Bereich des Instrumentenmauls und unter Ausblendung einzelner Bauteile und/oder Abschnitte,
- Fig. 3: in schematischer, teilweise abgeschnittener Längsschnittdarstellung das medizinische Instrument nach Fig. 1 im Bereich der Griffvorrichtung,
- Fig. 4: in perspektivischer Längsschnittdarstellung die Griffvorrichtung des medizinischen Instruments nach den Fig. 1 bis 3,
- Fig. 5: in schematischer Perspektivansicht ein der Griffvorrichtung zugeordnetes Sperrelement,
- Fig. 6, 7: teilweise abgeschnittene, schematische Längsschnittdarstellungen des medizinischen Instruments im Bereich der Griffvorrichtung, wobei ein beweglicher Handgriff der Griffvorrichtung eine unbetätigte Ruhestellung (Fig. 6) und eine betätigte Arbeitsstellung einnimmt (Fig. 7), und
- Fig. 8, 9, 10: jeweils in einer den Fig. 6 und 7 entsprechenden Darstellungsweise das medizinische Instrument in unterschiedlichen Konfigurationen zur Verdeutlichung einer Montage und/oder Demontage des Schafts an bzw. von der Griffvorrichtung.

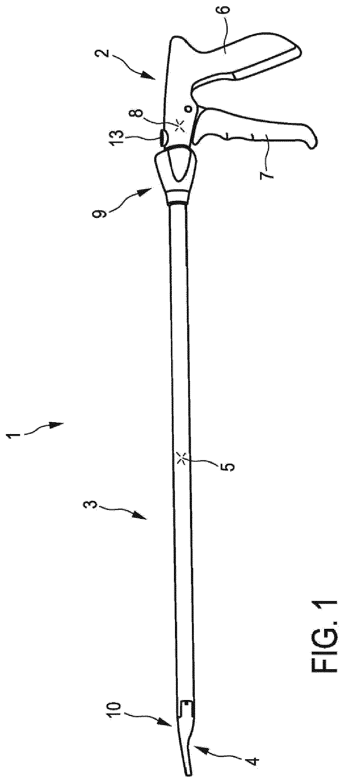

Gemäß Fig. 1 ist ein medizinisches Instrument zur Verwendung bei laparoskopischen Eingriffen vorgesehen. Vorliegend ist das medizinische Instrument eine medizinische Zange 1. Die medizinische Zange 1 dient einer Applikation medizinischer Clips und kann daher auch als medizinischer Clip-Applikator bezeichnet werden.

Die medizinische Zange 1 weist eine Griffvorrichtung 2, einen längserstreckten Schaft 3, ein Instrumentenmaul in Form eines Zangenmauls 4 und ein Zug-Druck-Element 5 auf.

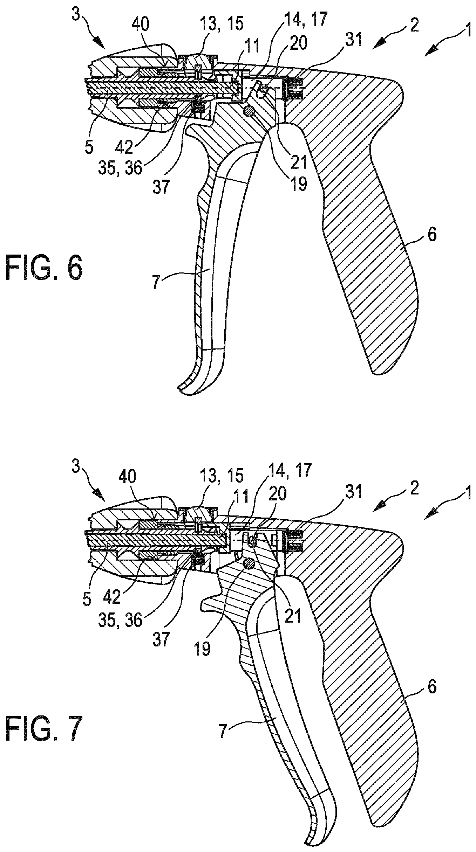

Die Griffvorrichtung 2 weist einen feststehenden Handgriff 6, einen beweglichen Handgriff 7 und eine Arretiereinrichtung 8 auf. Der bewegliche Handgriff 7 ist relativ zu dem feststehenden Handgriff 6 manuell zwischen einer nicht betätigten Ruhestellung (siehe Fig. 1, 3, 6) und einer betätigten Arbeitsstellung beweglich (siehe Fig. 7).

Der Schaft 3 ist zwischen einem proximalen Ende 9 und einem distalen Ende 10 längserstreckt. In der anhand Fig. 1 gezeigten Konfiguration nimmt die medizinische Zange 1 einen verwendungsbereiten Montagezustand ein. In diesem Montagezustand ist der Schaft 3 gemeinsam mit dem Zangenmaul 4 und dem Zug-Druck-Element 5 an der Griffvorrichtung 2 montiert. Dabei ist das proximale Ende 9 des Schafts 3 auf noch näher beschriebene Weise mittels der Arretiereinrichtung 8 lösbar an der Griffvorrichtung 2 arretiert. Die medizinische Zange 1 kann in einen Demontagezustand überführt werden, in welchem die Griffvorrichtung 2 von den übrigen Komponenten der medizinischen Zange 1 getrennt ist. Zu diesem Zweck ist die besagte Arretierung auf noch näher beschriebene Weise lösbar.

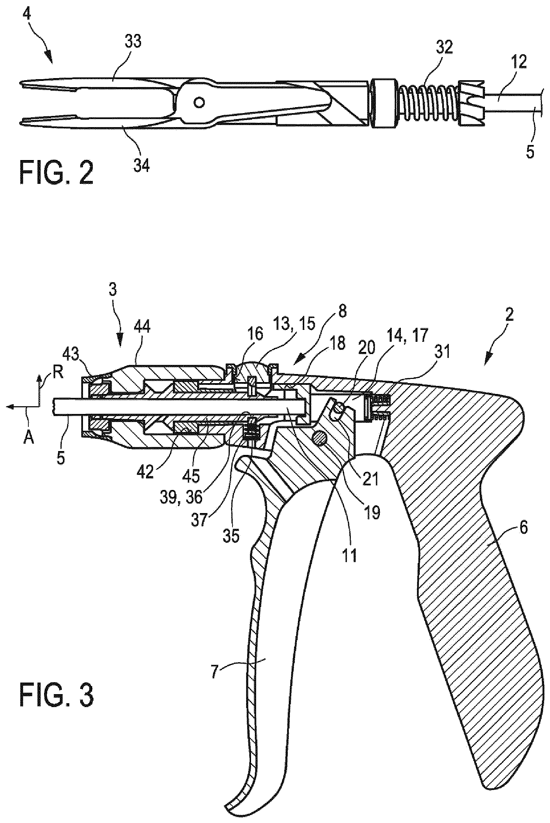

Das Zangenmaul 4 ist mittels einer Betätigung des beweglichen Handgriffs 7 zwischen einer geschlossenen Schließstellung (Fig. 1) und einer geöffneten Öffnungsstellung beweglich (Fig. 2). Dementsprechend sind das Zangenmaul 4 und der bewegliche Handgriff 7 zwecks Kraft- und Bewegungsübertragung miteinander wirkverbunden.

Die besagte Wirkverbindung erfolgt mittels des Zug-Druck-Elements 5. Das Zug-Druck-Element 5 ist zwischen einem proximalen Ende 11 (Fig. 3) und einem distalen Ende 12 längserstreckt (Fig. 2). Das proximale Ende 11 ist mit dem beweglichen Handgriff 7 wirkverbunden. Das distale Ende 12 ist mit dem Zangenmaul 4 wirkverbunden. Dabei ist das Zug-Druck-Element 5 axial beweglich in dem Schaft 3 geführt. Bei der gezeigten Ausführungsform sind der Schaft 3 und das Zug-Druck-Element 5 koaxial zueinander orientiert.

Zum Lösen der Arretierung weist die Arretiereinrichtung 8 ein Betätigungselement 13 auf. Das Betätigungselement 13 ist manuell zwischen einer nicht betätigten Ruhestellung (insbesondere Fig. 1, 3, 4) und einer zeichnerisch nicht näher dargestellten, betätigten Auslösestellung beweglich. In der Ruhestellung des Betätigungselements 13 ist die Arretierung aktiv. In der Auslösestellung des Betätigungselements 13 ist die Arretierung gelöst oder auch aufgehoben. Sobald die Arretierung gelöst ist, können die Griffvorrichtung 2 und der Schaft 3 zwecks Demontage voneinander getrennt werden.

Um zu vermeiden, dass die Arretierung in einem hierfür nicht vorgesehenen Zustand der medizinischen Zange 1 gelöst wird, weist die Arretiereinrichtung 8 ein Sperrelement 14 auf (siehe Fig. 3). Das Sperrelement 14 ist mit dem beweglichen Handgriff 7 wirkverbunden. Mit anderen Worten ausgedrückt, ist das Sperrelement 14 in Abhängigkeit der Bewegung des beweglichen Handgriffs 7 verlagerbar und/oder umgekehrt. In der Ruhestellung des beweglichen Handgriffs 7 nimmt das Sperrelement 14 eine Freigabestellung ein (siehe Fig. 3, 6). In der Arbeitsstellung des beweglichen Handgriffs 7 nimmt das Sperrelement 14 eine Sperrstellung ein (Fig. 7). In der Sperrstellung ist die Beweglichkeit des Betätigungselements 13 in die Auslösestellung mittels des Sperrelements 14 formschlüssig gesperrt. In der Freigabestellung ist die Beweglichkeit des Betätigungselements 13 von dem Sperrelement 14 unbeeinflusst und insoweit freigegeben. Hierdurch wird verhindert, dass die Arretierung in betätigtem Zustand der medizinischen Zange 1 gelöst wird.

Nachfolgend werden weitere strukturelle und funktionelle Merkmale der medizinischen Zange 1 erläutert. Die nachfolgend erläuterten Merkmale sind als vorteilhaft zu erachten. Im Hinblick auf die vorliegende Erfindung sind die nachfolgend erläuterten Merkmale jedoch nicht wesentlich.

Bei der gezeigten Ausführungsform ist das Betätigungselement 13 in Radialrichtung R des Zug-Druck-Elements 5 beweglich. Das Sperrelement 14 ist in Axialrichtung A des Zug-Druck-Elements 5 beweglich. Da bei der gezeigten Ausführungsform der Schaft 3 und das Zug-Druck-Element 5 koaxial zueinander orientiert sind, kann auch von der Radialrichtung R des Schafts 3 und dessen Axialrichtung A gesprochen werden. Bei einer zeichnerisch nicht dargestellten Ausführungsform können die Beweglichkeiten des Betätigungselements und des Sperrelements hierzu unterschiedlich sein. Beispielsweise kann das Sperrelement radial und das Betätigungselement axial beweglich sein.

Das Betätigungselement 13 ist in seiner nicht betätigten Ruhestellung radial nach außen und in seiner betätigten Auslösestellung radial nach innen verlagert. Das Sperrelement 14 ist in seiner Freigabestellung axial nach hinten (proximal) und in seiner Sperrstellung axial nach vorne (distal) verlagert. Bei einer zeichnerisch nicht dargestellten Ausführungsform ist dementgegen die Auslösestellung eine radial nach außen verlagerte Stellung des Betätigungselements. Dementsprechend kann die Sperrstellung eine proximal verlagerte Stellung des Sperrelements sein.

Bei der gezeigten Ausführungsform ist das Sperrelement 14 in der Sperrstellung wenigstens abschnittsweise unter das Betätigungselement 13 geschoben, so dass dieses in Radialrichtung R nicht nach innen bewegt werden kann (Fig. 7). Die formschlüssige Sperrung des Betätigungselements 13 wirkt demnach in Radialrichtung R.

Vorliegend ist das Betätigungselement 13 ein in die Auslösestellung radial eindrückbarer Druckknopf 15. Der Druckknopf 15 ist an einer Oberseite der Griffvorrichtung 2 angeordnet. Der Druckknopf 15 ist in einer Aufnahmeaussparung 16 der Griffvorrichtung 2 aufgenommen. Dabei ist der Druckknopf 15 in Radialrichtung R beweglich und senkrecht hierzu formschlüssig in der Aufnahmeaussparung 16 geführt.

Das Sperrelement 14 ist bei der gezeigten Ausführungsform ein linearbeweglicher Schieber 17. Der Schieber 17 ist in einer hierfür vorgesehenen, weiteren Aufnahmeaussparung 18 der Griffvorrichtung 2 aufgenommen.

Vorliegend sind sowohl das Betätigungselement 13 als auch das Sperrelement 14 beweglich an dem feststehenden Handgriff 6 gelagert.

Die Wirkverbindung zwischen dem beweglichen Handgriff 7 und dem Sperrelement 14 kann grundsätzlich auf unterschiedliche Weise gestaltet sein. Bei der gezeigten Ausführungsform ist der bewegliche Handgriff 7 um eine Schwenkachse 19 schwenkbeweglich an dem feststehenden Handgriff 6 gelagert und das Sperrelement 14 weist eine parallel zu der Schwenkachse 19 orientierte Mitnehmerachse 20 auf, die mit einer stirnendseitig in dem beweglichen Handgriff 7 eingebrachten Mitnehmernut 21 zusammenwirkt. In der Arbeitsstellung ist der bewegliche Handgriff 7 um die Schwenkachse 19 schwenkbeweglich an den feststehenden Handgriff 6 heranbewegt. In der Freigabestellung ist der bewegliche Handgriff 7 um die Schwenkachse 19 von dem feststehenden Handgriff 6 wegbewegt. Die Schwenkachse 19 ist orthogonal zu der Axialrichtung A und der Radialrichtung R orientiert. Mit anderen Worten ausgedrückt, ist die Schwenkachse 19 parallel zu einer nicht näher bezeichneten Querachse der Griffvorrichtung 2 und/oder des Schafts 3 orientiert. Entsprechendes gilt für die parallel zu der Schwenkachse 19 orientierte Mitnehmerachse 20 des Sperrelements 14. Die Mitnehmerachse 20 greift in Axialrichtung A formschlüssig in die Mitnehmernut 21 ein. Die Mitnehmernut 21 und die Mitnehmerachse 20 wirken in Umfangsrichtung der Mitnehmerachse 20 gleitbeweglich zusammen. Das Sperrelement 14 ist in Axialrichtung A gleitbeweglich und in Radialrichtung R formschlüssig in der weiteren Aufnahmeaussparung 18 gehalten. Hierdurch bewirkt eine Schwenkbewegung des Handgriffs 7 in Richtung der Arbeitsstellung eine distale Linearbewegung des Sperrelements 14.

Bei der gezeigten Ausführungsform ist der bewegliche Handgriff 7 ausschließlich mittels des Sperrelements 14 kraft- und bewegungsübertragend mit dem Zug-Druck-Element 5 wirkverbunden. Dementsprechend weist das Sperrelement 14 bei der gezeigten Ausführungsform eine besonders vorteilhafte Mehrfachfunktion auf. Zum einen dient das Sperrelement 14 der bereits beschriebenen Sperrung des Betätigungselements 13. Zum anderen dient das Sperrelement 14 zusätzlich als mechanisches Übertragungselement zum Übertragen der Betätigungsbewegung und/oder Betätigungskraft des beweglichen Handgriffs 7 auf das Zug-Druck-Element 5 und damit auf das Zangenmaul 4.

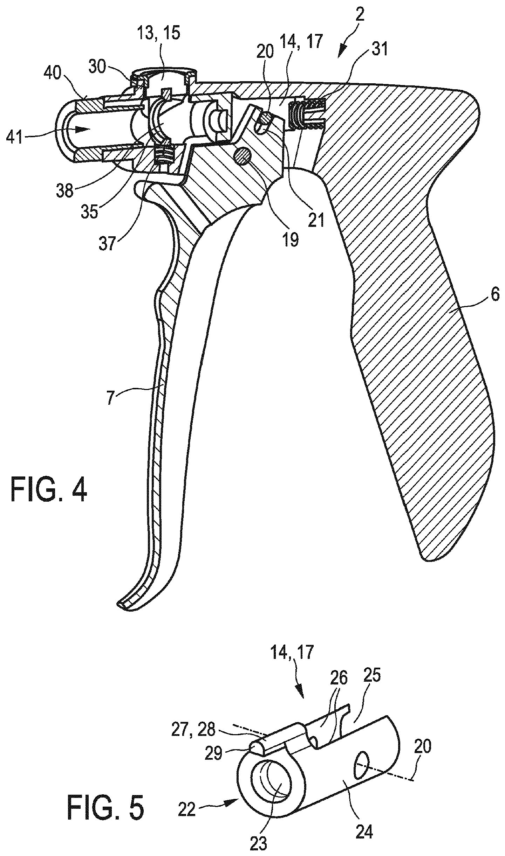

Zur Wirkverbindung mit dem Zug-Druck-Element 5 weist das Sperrelement 14 einen distal angeordneten Stützabschnitt 22 auf (Fig. 5). Das proximale Ende 11 des Zug-Druck-Elements 5 ist in Axialrichtung A an dem Stützabschnitt 22 abgestützt. Vorliegend ist das proximale Ende 11 zudem in Radialrichtung R formschlüssig an dem Stützabschnitt 22 aufgenommen. Zu diesem Zweck weist der Stützabschnitt 22 eine ausgehend von dem distalen Stirnende des Sperrelements 14 in proximaler Richtung erstreckte Einsenkung 23 auf. Das proximale Ende 11 ist in proximaler Richtung in die Einsenkung 23 lösbar eingesteckt.

Anhand Fig. 5 sind weitere Merkmale des Sperrelements 14 der vorliegenden Ausführungsform gezeigt. Das Sperrelement 14 weist vorliegend einen kreiszylindrischen Grundabschnitt 24 auf, der entlang der Axialrichtung A zwischen dem distalen Stützabschnitt 22 und einem nicht näher bezeichneten proximalen Stirnende längserstreckt ist. Der Grundabschnitt 24 weist eine in Radialrichtung R durchgängig zwischen einer Oberseite und einer Unterseite erstreckte Aussparung 25 auf. Das mit der Mitnehmernut 21 versehene, obere Stirnende des beweglichen Handgriffs 7 greift in Radialrichtung R in die Aussparung 25 ein und ist in Querrichtung zwischen einander gegenüberliegenden Seitenwandungen 26 des Grundabschnitts 24 angeordnet. Zur formschlüssigen Sperrung des Betätigungselements 13 weist das Sperrelement 14 einen Sperrabschnitt 27 auf. Der Sperrabschnitt 27 kann auch als Formschlussabschnitt bezeichnet werden. Der Sperrabschnitt 27 ragt in distaler Richtung von dem Grundabschnitt 24 ab. Der Sperrabschnitt 27 ist vorliegend ein distal abragender Sperrfinger 28. In der Sperrstellung untergreift der Sperrabschnitt 27 das Betätigungselement 13 und blockiert auf diese Weise eine Bewegung des Betätigungselements 13 in die Auslösestellung. Die Mitnehmerachse 20 ist in Querrichtung durch den Grundabschnitt 24 und/oder die Aussparung 25 erstreckt.

Bei der gezeigten Ausführungsform ist das Sperrelement 14 einstückig aus einem Kunststoffwerkstoff gefertigt. Alternativ kann das Sperrelement aus Metall und/oder mehrteilig gefertigt sein.

Beispielsweise anhand der Fig. 3 und 4 ist erkennbar, dass die Beweglichkeit des Sperrelements 14 - und damit auch des beweglichen Handgriffs 7 - in der radial nach innen verlagerten Auslösestellung des Betätigungselements 13 gesperrt ist. Denn in der nicht näher gezeigten Auslösestellung ist das Betätigungselement 13 derart radial nach innen verlagert, dass das Sperrelement 14 formschlüssig an einer Verlagerung in distaler Richtung gehindert ist. In der Auslösestellung fungiert ein Außenumfang 30 des Betätigungselements 13 als Anschlag für das Sperrelement 14, genauer für ein Stirnende 29 des Sperrabschnitts 27. Demnach weist auch das Betätigungselement 13 eine besonders vorteilhafte Mehrfachfunktion auf. Zum einen dient das Betätigungselement 13 der Betätigung der Arretiereinrichtung 8 zum Lösen der Arretierung. Zum anderen fungiert das Betätigungselement 13 zusätzlich als (weiteres) Sperrelement zur formschlüssigen Sperrung der Beweglichkeit des Sperrelements 14 und damit auch des beweglichen Handgriffs 7. Hierdurch wird verhindert, dass der bewegliche Handgriff 7 während der Betätigung des Betätigungselements 13, d.h. beim Lösen der Arretierung, betätigt werden kann.

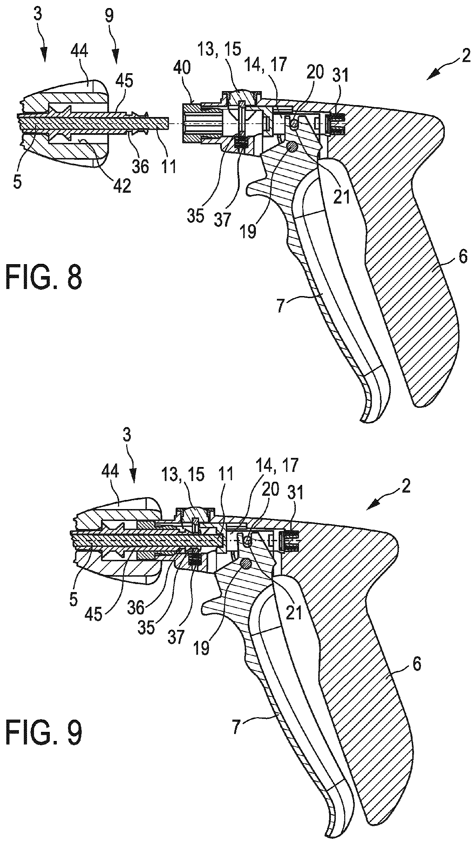

Bei der gezeigten Ausführungsform ist das Sperrelement 14 mittels eines an der Griffvorrichtung 2 angeordneten ersten Federelements 31 in Richtung der Sperrstellung federbelastet vorgespannt. Infolge dieser Vorspannung ist das Sperrelement 14 - in dem demontierte Demontagezustand der medizinischen Zange 1 (siehe Fig. 8) - stets in die Sperrstellung verlagert. Aufgrund der bestehenden Wirkverbindung mit dem Sperrelement 14 ist der bewegliche Handgriff 7 mittels des ersten Federelements 31 mittelbar federbelastet vorgespannt. Diese Vorspannung wirkt in Richtung der Arbeitsstellung. Folglich ist der bewegliche Handgriff 7 in dem Demontagezustand stets in die Arbeitsstellung verlagert (siehe Fig. 8). Das erste Federelement 31 wirkt in Axialrichtung A auf das Sperrelement 14. Das erste Federelement 31 ist in proximaler Richtung an dem feststehenden Handgriff 6 und in distaler Richtung an einem nicht näher bezeichneten proximalen Stirnende des Sperrelements 14 abgestützt. Bei der gezeigten Ausführungsform ist das erste Federelement 31 eine Druckfeder. Das Federelement 31 ist als Schraubenfeder ausgebildet.

Zur Vollständigkeit sei angemerkt, dass die Vorspannung des Sperrelements 14 und des beweglichen Handgriffs 7 in Fig. 4 zeichnerisch nicht umgesetzt ist. Mit anderen Worten ausgedrückt, ist der bewegliche Handgriff 7 zeichnerisch entgegen der Vorspannung des ersten Federelements 31 in der Ruhestellung festgehalten. Entsprechendes gilt sinngemäß für das Sperrelement 14.

Die besagte Vorspannung mittels des ersten Federelements 31 verhindert, dass das Betätigungselement 13 in dem Demontagezustand in die Auslösestellung verlagert werden kann. Hierdurch wird eine fehlerfreie Montage des Schafts 3 an der Griffvorrichtung 2 unterstützt, was nachfolgend noch näher beschrieben wird.

Sofern der Schaft 3 und die Griffvorrichtung 2 miteinander verbunden sind, d.h. die medizinische Zange 1 den Montagezustand einnimmt (Fig. 1, 3, 6, 7), wirkt zusätzlich zu dem ersten Federelement 31 ein zweites Federelement 32 auf das Sperrelement 14. Das zweite Federelement 32 (Fig. 2) bewirkt eine federbelastete Vorspannung des Sperrelements 14 in Richtung der Freigabestellung. Dabei ist das zweite Federelement 32 stärker dimensioniert als das erste Federelement 31. Mit anderen Worten ausgedrückt, überwiegt die Vorspannung des Sperrelements 14 in Richtung der Freigabestellung. Hierdurch ist das Sperrelement 14 in dem Montagezustand - und bei nicht vorhandener Betätigung des beweglichen Handgriffs 7 - stets in die Freigabestellung verlagert. Der bewegliche Handgriff 7 ist aufgrund der Wirkverbindung mit dem Sperrelement 14 mittels des zweiten Federelements 32 mittelbar federbelastet vorgespannt. Die Vorspannung wirkt in Richtung der Ruhestellung. Dies trotz der ebenfalls vorhandenen Vorspannung mittels des ersten Federelements 31.

Das zweite Federelement 32 ist an dem Schaft 3 angeordnet. Dementsprechend wirkt das zweite Federelement 32 nur dann auf das Sperrelement 14, wenn der Schaft 3 an der Griffvorrichtung 2 montiert ist.

Bei der gezeigten Ausführungsform fungiert das zweite Federelement 32 gleichzeitig als Rückstellfeder für das Zug-Druck-Element 5. Dabei wirkt das zweite Federelement 32 mittelbar über das Zug-Druck-Element 5 auf das Sperrelement 14. Das zweite Federelement 32 ist an dem distalen Ende 10 des Schafts 3 angeordnet. Das zweite Federelement 32 wirkt in Axialrichtung A auf das Zug-Druck-Element 5 und damit auch auf das Sperrelement 14. Das zweite Federelement 32 ist in proximaler Richtung an dem distalen Ende 12 des Zug-Druck-Elements abgestützt. Diese Abstützung kann mittelbar oder unmittelbar sein. In distaler Richtung ist das zweite Federelement 32 an einem nicht näher bezeichneten, dem Zangenmaul 4 zugeordneten Bauteil abgestützt. Die konstruktiven Details des Zangenmauls 4 sind hinsichtlich der Funktion des zweiten Federelements 32 nicht wesentlich, so dass weitere diesbezügliche Erläuterungen entbehrlich sind. Das zweite Federelement 32 ist vorliegend eine Druckfeder. Das zweite Federelement 32 ist als Schraubenfeder gestaltet.

Wie weiter anhand Fig. 2 gezeigt ist, ist das Zangenmaul 4 bei der gezeigten Ausführungsform zwischen einem ersten Maulteil 33 und einem zweiten Maulteil 34 gebildet. In der Öffnungsstellung (Fig. 2) sind das erste Maulteil 33 und das zweite Maulteil 34 voneinander wegbewegt. In der Schließstellung (Fig. 1) sind die beiden Maulteile 33, 34 aufeinander zubewegt.

Das Öffnen und/oder Schließen des Zangenmauls 4 erfolgt unter Einwirkung des Zug-Druck-Elements 5 und auf eine dem Fachmann grundsätzlich bekannte Weise. Dabei bewirkt eine distale Verlagerung des Zug-Druck-Elements 5 vorliegend ein Schließen des Zangenmauls 4. Eine proximale Verlagerung des Zug-Druck-Elements bewirkt ein Öffnen des Zangenmauls 5. Weitere diesbezügliche Erläuterungen sind im Hinblick auf die vorliegende Erfindung entbehrlich.

Zur Arretierung des Schafts 3 an der Griffvorrichtung 2 weist die Arretiervorrichtung 8 bei der gezeigten Ausführungsform eine federbelastete Klinke 35 auf. Zum Zusammenwirken mit der Klinke 35 weist der Schaft 3 an seinem proximalen Ende 9 eine Kerbe 36 auf. In dem Montagezustand und in der nicht betätigten Ruhestellung des Betätigungselements 13 greift die Klinke 35 in Axialrichtung A formschlüssig in die Kerbe 36 ein. Mittels einer Betätigung des Betätigungselements 13 kann die Klinke 35 in Radialrichtung R aus der Kerbe 36 ausgerückt werden. Dabei ist die Klinke 35 vorliegend federbelastet in Radialrichtung R nach innen vorgespannt. Zu diesem Zweck ist ein drittes Federelement 37 vorhanden. Das dritte Federelement 37 wirkt über die Klinke 35 mittelbar auf das Betätigungselement 13. Das Betätigungselement 13 ist mittels des dritten Federelements 37 entgegen einer Bewegung in die Auslösestellung federbelastet vorgespannt.

Die Kerbe 36 ist bei der gezeigten Ausführungsform eine in Umfangsrichtung des Schafts 3 erstreckte Ringnut 39. Die Ringnut 39 ist an dem proximalen Ende 11 des Schafts 3 ausgebildet. Im Übrigen weist die Klinke 35 eine Durchgangsbohrung 38 auf, durch welche das Zug-Druck-Element 5 hindurcherstreckt ist.

Die Arretierung, genauer: die Klinke-Kerbe-Verbindung zwischen der Klinke 35 und der Kerbe 36, wirkt einem ungewollten Lösen des Schafts 3 von der Griffvorrichtung 2 und umgekehrt entgegen. Zur Verbindung mit dem Schaft 3 weist die Griffvorrichtung 2 vorliegend einen distal angeordneten Außenkonus 40 mit einer Aufnahmebohrung 41 auf. Zur Verbindung mit der Griffvorrichtung 2 weist der Schaft 3 einen Innenkonus 42 auf. In dem Montagezustand sind der Außenkonus 40 und der Innenkonus 42 axial lösbar zusammengesteckt. Die hierdurch ausgebildete Steckverbindung ist mittels der Arretiereinrichtung 8 lösbar arretiert.

Der Schaft 3 ist bei der gezeigten Ausführungsform mehrteilig aufgebaut und weist ein Schaftrohr 43, einen Schaftansatz 44 und einen Schaftfortsatz 45 auf (Fig. 3). Das Schaftrohr 43, der Schaftansatz 44 und der Schaftfortsatz 45 sind auf eine dem Fachmann bekannte Weise zusammengefügt. Vorliegend ist der Innenkonus 42 an dem Schaftansatz 44 ausgebildet. Die Kerbe 36 ist an dem Schaftfortsatz 45 ausgebildet. Der Schaftfortsatz 45 ist in dem Montagezustand in die Aufnahmebohrung 41 eingesteckt und durch die Durchgangsbohrung 38 hindurcherstreckt. Es versteht sich, dass die vorliegende Gestaltung des Schafts 3 exemplarisch und im Hinblick auf die Erfindung nicht wesentlich ist. Denn grundsätzlich ist auch eine einteilige Gestaltung des Schafts denkbar.

Nachfolgend wird anhand der Fig. 6 bis 10 die Funktion der medizinischen Zange 1 im Detail erläutert. Insbesondere wird die Funktion des Sperrelements 14 und deren Zusammenwirken mit den weiteren Komponenten der medizinischen Zange 1 beschrieben. Zunächst wird der Montagezustand betrachtet (Fig. 6, 7). Hiernach die Montage/Demontage (Fig. 8, 9, 10).

Fig. 6 zeigt die medizinische Zange 1 in dem Montagezustand und in einer ersten Konfiguration, die auch als nicht betätigte Konfiguration bezeichnet werden kann. In der ersten Konfiguration nimmt der bewegliche Handgriff 7 seine Ruhestellung ein. Das zweite Federelement 32 wirkt über das Zug-Druck-Element 5 in proximaler Richtung auf das Sperrelement 14. Das erste Federelement 31 wirkt in distaler Richtung auf das Sperrelement 14. Da die Federkraft des zweiten Federelements 32 größer ist als die Federkraft des ersten Federelements 31, überwiegt die federbelastete Vorspannung in proximaler Richtung. Folglich ist das Sperrelement 14 in die Freigabestellung verlagert/vorgespannt. Der bewegliche Handgriff 7 ist in die Ruhestellung verlagert/vorgespannt. Die Arretierung ist aktiv. Dabei ist die Klinke 35 mittels des dritten Federelements 37 in die Kerbe 36 eingerückt. Das dritte Federelement 37 bewirkt mittelbar, nämlich über die Klinke 35, eine Vorspannung des Betätigungselements 13 entgegen einer Bewegung in die Auslösestellung.

Ausgehend von der ersten Konfiguration kann das Betätigungselement 13 in die Auslösestellung verlagert werden. Hierdurch wird die Klinke 35 in radialer Richtung R nach außen aus der Kerbe 36 ausgerückt. Dies bewirkt ein Lösen der Arretierung. Der Schaft 3 kann hiernach in distaler Richtung von der Griffvorrichtung 2 abgezogen werden. Hierdurch wird die medizinische Zange 1 demontiert und/oder in ihren Demontagezustand überführt (Fig. 8).

Der Demontagezustand (Fig. 8) kann auch als dritte Konfiguration bezeichnet werden. In dieser dritten Konfiguration bewirkt das erste Federelement 31 eine Verlagerung des Sperrelements 14 in die Sperrstellung. Der bewegliche Handgriff 7 ist dementsprechend in seine Arbeitsstellung verlagert. Die Beweglichkeit des Betätigungselements 13 ist mittels des Sperrelements 14 formschlüssig gesperrt.

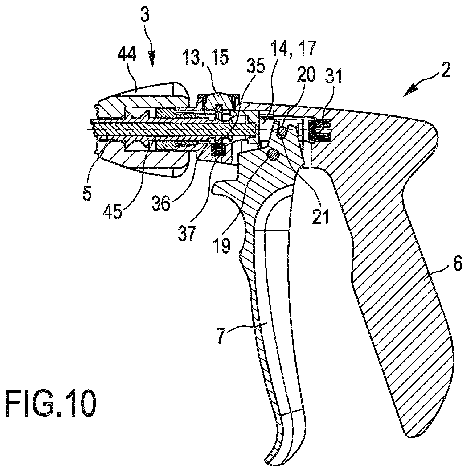

Ausgehend von der dritten Konfiguration (Fig. 8) kann der Schaft erneut an der Griffvorrichtung 2 montiert werden. Eine solche Montage ist anhand der Fig. 9 und 10 verdeutlicht. Zur Montage wird das proximale Ende 9 des Schafts 3 in Axialrichtung mit der Griffvorrichtung 2 zusammengesteckt. Hierdurch wird die vorbeschriebene Steckverbindung zwischen dem Außenkonus 40 und dem Innenkonus 42 hergestellt.

Fig. 9 zeigt eine vierte Konfiguration, in welcher der Schaft 3 und die Griffvorrichtung 2 ausgehend von der dritten Konfiguration teilweise zusammengesteckt sind. In dieser vierten Konfiguration ist der Schaftfortsatz 45 zusammen mit dem Zug-Druck-Element 5 in proximaler Richtung in die Aufnahmebohrung 41 eingesteckt. Die Klinke-Kerbe-Verbindung zwischen der Klinke 35 und der Kerbe 36 ist noch nicht etabliert. Dementsprechend besteht noch keine Arretierung der (teilweisen) Steckverbindung. Das proximale Ende 11 des Zug-Druck-Elements 5 ist bereits an dem Stützabschnitt 22 aufgenommen.

Fig. 10 zeigt eine fünfte Konfiguration, bei welcher der Schaft 3 und die Griffvorrichtung 2 weiter zusammengesteckt sind. Dabei ist der Schaft 3 zusammen mit dem Zug-Druck-Element 5 relativ zu der Griffvorrichtung 2 weiter in proximaler Richtung verlagert. Hierdurch ist das Sperrelement 14 unter Einwirkung des proximalen Endes 11 des Zug-Druck-Elements 5 in proximaler Richtung, d.h. in Richtung seiner Freigabestellung, gedrückt. Der bewegliche Handgriff 7 ist dementsprechend bereits teilweise in Richtung seiner Ruhestellung verlagert. Das Betätigungselement 14 ist mittels des Sperrelements 14 nach wie vor formschlüssig gesperrt. Die Klinke 35 ist noch nicht in die Kerbe 36 eingerückt.

Durch ein weitergehendes Zusammenstecken des Schafts 3 und der Griffvorrichtung 2 wird ausgehend von der fünften Konfiguration die erste Konfiguration (Fig. 6) und damit der Montagezustand erreicht.

Ausgehend von dem Montagezustand und/oder der ersten Konfiguration kann die medizinische Zange 1 zum Öffnen und/oder Schließen des Zangenmauls 4 betätigt werden (Fig. 7, zweite Konfiguration). Vorliegend bewirkt eine Verlagerung des beweglichen Handgriffs 7 ausgehend von der Ruhestellung in die Arbeitsstellung ein Schließen des Zangenmauls 4. Ein Heranziehen des beweglichen Handgriffs 7 an den feststehenden Handgriff 6 bewirkt, dass der bewegliche Handgriff 7 entgegen dem Uhrzeigersinn um die Schwenkachse 19 verlagert wird. Aufgrund des Zusammenwirkens der Mitnehmernut 21 und der Mitnehmerachse 20 wird hierdurch gleichzeitig das Sperrelement 14 in distale Richtung verlagert. Hierdurch wird zum einen die Beweglichkeit des Betätigungselements 13 in Richtung der Auslösestellung formschlüssig blockiert. Zum anderen drückt das Sperrelement 14 das Zug-Druck-Element 5 in distale Richtung. Hierdurch wird das Zug-Druck-Element 5 relativ zu dem Schaft 3 und dem distal an dem Schaft 3 montierten Zangenmaul 4 axial beweglich verlagert. Das distale Ende 12 wirkt kraft- und bewegungsübertragend auf das Zangenmaul 4, so dass letzteres geschlossen wird. Die axiale Bewegung des Zug-Druck-Elements 5 in distale Richtung erfolgt entgegen der Federkraft des zweiten Federelements 32. Sobald die Betätigung des beweglichen Handgriffs 7 aufgehoben wird, bewirkt das zweite Federelement 32 eine proximal gerichtete Rückstellbewegung des Zug-Druck-Elements 5, ein Öffnen des Zangenmauls 4, eine Verlagerung des Sperrelements 14 in die Freigabestellung und eine Verlagerung des beweglichen Handgriffs 7 in die Ruhestellung. Hiernach wird erneut die erste Konfiguration eingenommen.

## Patentansprüche

1. Medizinisches Instrument (1), insbesondere zur Applikation medizinischer Clips, aufweisend
eine Griffvorrichtung (2) mit einem feststehenden Handgriff (6), einem beweglichen Handgriff (7), der relativ zu dem feststehenden Handgriff (6) manuell zwischen einer nicht betätigten Ruhestellung und einer betätigten Arbeitsstellung beweglich ist, und mit einer Arretiereinrichtung (8),
einen längserstreckten Schaft (3), dessen proximales Ende (9) mittels der Arretiereinrichtung (8) lösbar an der Griffvorrichtung (2) arretiert ist,
ein an einem distalen Ende (10) des Schafts (3) angeordnetes Instrumentenmaul (4), das mittels einer Betätigung des beweglichen Handgriffs (7) zwischen einer Öffnungsstellung und einer Schließstellung beweglich ist,
und aufweisend ein längserstrecktes Zug-Druck-Element (5), das axial beweglich in dem Schaft (3) geführt und zur Kraft- und Bewegungsübertragung proximal mit dem beweglichen Handgriff (7) und distal mit dem Instrumentenmaul (4) wirkverbunden ist,
**dadurch gekennzeichnet, dass** die Arretiereinrichtung (8) ein Betätigungselement (13), das zum Lösen der Arretierung manuell in eine Auslösestellung beweglich ist, und ein mit dem beweglichen Handgriff (7) wirkverbundenes Sperrelement (14) aufweist, das in der Arbeitsstellung des beweglichen Handgriffs (7) in eine Sperrstellung verlagert ist, in welcher das Sperrelement (14) die Beweglichkeit des Betätigungselements (13) formschlüssig sperrt, und das in der Ruhestellung des beweglichen Handgriffs (7) in eine Freigabestellung verlagert ist, in welcher die Beweglichkeit des Betätigungselements (13) freigegeben ist.

2. Medizinisches Instrument (1) nach Anspruch 1, **dadurch gekennzeichnet, dass** das Betätigungselement (13) in der Auslösestellung die Beweglichkeit des Sperrelements (14) in die Sperrstellung und/oder die Beweglichkeit des beweglichen Handgriffs (7) in die Arbeitsstellung sperrt.

3. Medizinisches Instrument (1) nach Anspruch 1 oder 2, **dadurch gekennzeichnet, dass** das Sperrelement (14) mittels eines an der Griffvorrichtung (2) angeordneten ersten Federelements (31) in Richtung der Sperrstellung federbelastet vorgespannt ist, und - in dem arretierten Zustand des Schafts (3) - mittels eines an dem Schaft (3) angeordneten zweiten Federelements (32) in Richtung der Freigabestellung federbelastet vorgespannt ist, wobei das zweite Federelement (32) eine stärkere Vorspannung bewirkt als das erste Federelement (31).

4. Medizinisches Instrument (1) nach einem der vorhergehenden Ansprüche, **dadurch gekennzeichnet, dass** das Betätigungselement (13) in Radialrichtung (R) des Zug-Druck-Elements (5) beweglich ist, und dass das Sperrelement (14) in Axialrichtung (A) des Zug-Druck-Elements (5) beweglich ist.

5. Medizinisches Instrument (1) nach einem der vorhergehenden Ansprüche, **dadurch gekennzeichnet, dass** das Betätigungselement (13) ein in die Auslösestellung eindrückbarer Druckknopf (15) ist, und dass das Sperrelement (14) ein linearbeweglicher Schieber (17) ist, der in der Sperrstellung wenigstens abschnittsweise unter den Druckknopf (15) geschoben ist.

6. Medizinisches Instrument (1) nach einem der vorhergehenden Ansprüche, **dadurch gekennzeichnet, dass** der bewegliche Handgriff (7) um eine Schwenkachse (19) schwenkbeweglich an dem feststehenden Handgriff (6) gelagert ist, und dass das Sperrelement (14) eine parallel zu der Schwenkachse (19) orientierte Mitnehmerachse (20) aufweist, die mit einer stirnendseitig in den beweglichen Handgriff (7) eingebrachten Mitnehmernut (21) zusammenwirkt.

7. Medizinisches Instrument (1) nach einem der vorhergehenden Ansprüche, **dadurch gekennzeichnet, dass** der bewegliche Handgriff (7) mittels des Sperrelements (14) kraft- und bewegungsübertragend auf ein proximales Ende (11) des Zug-Druck-Elements (5) wirkt.

8. Medizinisches Instrument (1) nach Anspruch 7, **dadurch gekennzeichnet, dass** das Sperrelement (14) einen distal angeordneten Stützabschnitt (22) aufweist, an welchem das proximale Ende (11) des Zug-Druck-Elements (5) in Axialrichtung (A) abgestützt und/oder in Radialrichtung (R) formschlüssig aufgenommen ist.

9. Medizinisches Instrument (1) nach einem der vorhergehenden Ansprüche, **dadurch gekennzeichnet, dass** die Arretiereinrichtung (8) eine federbelastete Klinke (35) aufweist, die in Axialrichtung (A) formschlüssig in eine Kerbe (36) des Schafts (3) eingreift und mittels des Betätigungselements (13) aus der Kerbe (36) ausrückbar ist.

## Claims

1. Medical instrument (1), in particular for applying medical clips, having
a handle device (2) with a fixed handle part (6), a movable handle part (7) which is manually movable relative to the fixed handle part (6) between a non-actuated rest position and an actuated working position, and with a locking device (8),
an elongate shaft (3) whose proximal end (9) is releasably locked on the handle device (2) by means of the locking device (8),
an instrument jaw (4) which is arranged on a distal end (10) of the shaft (3) and which is movable between an open position and a closed position by an actuation of the movable handle part (7),
and having an elongate push-pull element (5) which is guided axially movably in the shaft (3) and which, for force and movement transmission, is operatively connected proximally to the movable handle part (7) and distally to the instrument jaw (4),
**characterized in that** the locking device (8) has an actuation element (13) which is movable manually into a release position in order to release the locking, and a blocking element (14) which is operatively connected to the movable handle part (7) and which is moved into a blocking position in the working position of the movable handle part (7), in which blocking position the blocking element (14) interlockingly blocks the mobility of the actuation element (13), and which is moved into an enabling position in the rest position of the movable handle part (7), in which enabling position the mobility of the actuation element (13) is enabled.

2. Medical instrument (1) according to Claim 1, **characterized in that** the actuation element (13) in the release position blocks the mobility of the blocking element (14) into the blocking position and/or the mobility of the movable handle part (7) into the working position.

3. Medical instrument (1) according to Claim 1 or 2, **characterized in that** the blocking element (14) is pretensioned with spring loading in the direction of the blocking position by a first spring element (31) arranged on the handle device (2), and, in the locked state of the shaft (3), is pretensioned with spring loading in the direction of the enabling position by a second spring element (32) arranged on the shaft (3), wherein the second spring element (32) causes greater pretensioning than the first spring element (31).

4. Medical instrument (1) according to one of the preceding claims, **characterized in that** the actuation element (13) is movable in the radial direction (R) of the push-pull element (5), and **in that** the blocking element (14) is movable in the axial direction (A) of the push-pull element (15).

5. Medical instrument (1) according to one of the preceding claims, **characterized in that** the actuation element (13) is a push button (15) which is able to be pressed into the release position, and **in that** the blocking element (14) is a linearly movable slide (17) which, in the blocking position, is slid at least partially under the push button (15).

6. Medical instrument (1) according to one of the preceding claims, **characterized in that** the movable handle part (7) is mounted on the fixed handle part (6) pivotably about a pivot pin (19), and **in that** the blocking element (14) has a driver pin (20) which is oriented parallel to the pivot pin (19) and which interacts with a driver groove (21) introduced into the end face of the movable handle part (7).

7. Medical instrument (1) according to one of the preceding claims, **characterized in that** the movable handle part (7) acts on a proximal end (11) of the push-pull element (5) in a force-transmitting and movement-transmitting manner by way of the blocking element (14).

8. Medical instrument (1) according to Claim 7, **characterized in that** the blocking element (14) has a distally arranged supporting portion (22) on which the proximal end (11) of the push-pull element (5) is supported in the axial direction (A) and/or is received interlockingly in the radial direction (R).

9. Medical instrument (1) according to one of the preceding claims, **characterized in that** the locking device (8) has a spring-loaded latch (35) which in the axial direction (A) engages interlockingly in a notch (36) of the shaft (3) and can be disengaged from the notch (36) by the actuation element (13).

## Revendications

1. Instrument médical (1), notamment destiné à l'application de clips médicaux, comprenant
un dispositif de préhension (2) comportant une poignée fixe (6), une poignée mobile (7), qui peut être déplacée manuellement par rapport à la poignée fixe (6) entre une position de repos non actionnée et une position de travail actionnée, et comportant un appareil de blocage (8),
une tige allongée (3), dont l'extrémité proximale (9) est bloquée de manière amovible sur le dispositif de préhension (2) au moyen de l'appareil de blocage (8),
une mâchoire d'instrument (4) disposée au niveau d'une extrémité distale (10) de la tige (3), laquelle peut être déplacée entre une position d'ouverture et une position de fermeture par actionnement de la poignée mobile (7),
et un élément de traction-compression allongé (5), qui est guidé de manière axialement mobile dans la tige (3) et est relié fonctionnellement, à son extrémité proximale, à la poignée mobile (7) et, à son extrémité distale, à la mâchoire d'instrument (4), afin de transmettre une force et un mouvement,
**caractérisé en ce que** l'appareil de blocage (8) comporte un élément d'actionnement (13), qui peut être déplacé manuellement dans une position de déclenchement afin de libérer le blocage, et un élément de verrouillage (14) relié fonctionnellement à la poignée mobile (7), lequel, dans la position de travail de la poignée mobile (7), est déplacé dans une position de verrouillage dans laquelle l'élément de verrouillage (14) bloque par complémentarité de forme la mobilité de l'élément d'actionnement (13), et, dans la position de repos de la poignée mobile (7), est déplacé dans une position de libération dans laquelle la mobilité de l'élément d'actionnement (13) est libérée.

2. Instrument médical (1) selon la revendication 1, **caractérisé en ce que** l'élément d'actionnement (13), dans la position de déclenchement, bloque le déplacement de l'élément de verrouillage (14) vers la position de verrouillage et/ou le déplacement de la poignée mobile (7) vers la position de travail.

3. Instrument médical (1) selon la revendication 1 ou 2, **caractérisé en ce que** l'élément de verrouillage (14) est précontraint élastiquement en direction de la position de verrouillage au moyen d'un premier élément élastique (31) disposé sur le dispositif de préhension (2) et, dans l'état bloqué de la tige (3), est précontraint élastiquement en direction de la position de libération au moyen d'un second élément élastique (32) disposé sur la tige (3), le second élément élastique (32) produisant une précontrainte supérieure à celle du premier élément élastique (31).

4. Instrument médical (1) selon l'une des revendications précédentes, **caractérisé en ce que** l'élément d'actionnement (13) est mobile dans la direction radiale (R) de l'élément de traction-compression (5), et **en ce que** l'élément de verrouillage (14) est mobile dans la direction axiale (A) de l'élément de traction-compression (5).

5. Instrument médical (1) selon l'une des revendications précédentes, **caractérisé en ce que** l'élément d'actionnement (13) est un bouton-poussoir (15) pouvant être enfoncé dans la position de déclenchement, et **en ce que** l'élément de verrouillage (14) est un coulisseau (17) mobile linéairement qui, dans la position de verrouillage, est glissé au moins par sections sous le bouton-poussoir (15).

6. Instrument médical (1) selon l'une des revendications précédentes, **caractérisé en ce que** la poignée mobile (7) est montée pivotante autour d'un axe de pivotement (19) sur la poignée fixe (6), et **en ce que** l'élément de verrouillage (14) comporte un axe d'entraînement (20) orienté parallèlement à l'axe de pivotement (19), lequel coopère avec une rainure d'entraînement (21) ménagée dans une face d'extrémité de la poignée mobile (7).

7. Instrument médical (1) selon l'une des revendications précédentes, **caractérisé en ce que** la poignée mobile (7) agit, par l'intermédiaire de l'élément de verrouillage (14), sur une extrémité proximale (11) de l'élément de traction-compression (5) de manière à transmettre une force et un mouvement.

8. Instrument médical (1) selon la revendication 7, **caractérisé en ce que** l'élément de verrouillage (14) comporte une section de support (22) disposée distalement, contre laquelle l'extrémité proximale (11) de l'élément de traction-compression (5) prend appui dans la direction axiale (A) et/ou dans laquelle elle est reçue par complémentarité de forme dans la direction radiale (R).

9. Instrument médical (1) selon l'une des revendications précédentes, **caractérisé en ce que** l'appareil de blocage (8) comporte un cliquet (35) sollicité élastiquement, qui vient en prise par complémentarité de forme dans une encoche (36) de la tige (3) dans la direction axiale (A) et qui peut être dégagé de l'encoche (36) au moyen de l'élément d'actionnement (13).
